# FASCICULE DE BREVET EUROPEEN

(11) **EP 4 303 566 B1**
(45) Date de publication et mention de la délivrance du brevet: **09.12.2026**
(21) Numéro de dépôt: 23184075.2
(22) Date de dépôt: 07.07.2023
(51) Int. Cl.: G01N 21/3577, C12Q 1/04

(54) **MEMBRANE EN SILICIUM À TRANSMITTANCE DANS L'INFRAROUGE ET SON PROCÉDÉ DE FABRICATION**
SILIZIUMMEMBRAN MIT INFRAROTDURCHLÄSSIGKEIT UND VERFAHREN ZU IHRER HERSTELLUNG
SILICON MEMBRANE HAVING INFRARED TRANSMITTANCE AND METHOD FOR PRODUCING SAME

(30) Priorité: 08.07.2022 FR 2207005
(43) Date de publication de la demande: 10.01.2024
(73) Titulaire: Commissariat à l'Energie Atomique et aux Energies Alternatives, 75015 Paris (FR)
(72) Inventeur: GAILLARD, Frederic-Xavier, 38054 Grenoble cedex 09 (FR); OTSUKA, Yoko, 38054 Grenoble cedex 09 (FR)
(74) Mandataire: Ipsilon

(56) Documents cités:
- WO-A1-2015/044308
- FR-A1- 3 108 983
- BAO ET AL: "The complex correlation between current density and pore density based on non-SCR effects", ELECTROCHEMISTRY COMMUNICATIONS, ELSEVIER AMSTERDAM, NL, vol. 9, no. 8, 31 May 2007 (2007-05-31), pages 1991 - 1997, XP022158319, ISSN: 1388-2481, DOI: 10.1016/J.ELECOM.2007.05.014
- LASCAUD J ET AL: "Integrating porous silicon layer backing to capacitive micromachined ultrasonic transducers (CMUT)-based linear arrays for acoustic Lamb wave attenuation", JOURNAL OF APPLIED PHYSICS, AMERICAN INSTITUTE OF PHYSICS, 2 HUNTINGTON QUADRANGLE, MELVILLE, NY 11747, vol. 131, no. 10, 11 March 2022 (2022-03-11), XP012264147, ISSN: 0021-8979, [retrieved on 20220311], DOI: 10.1063/5.0083052
- VAN DEN BERG A ET AL: "A micro-volume open liquid-junction reference electrode for pH-ISFETs", SENSORS AND ACTUATORS B: CHEMICAL, ELSEVIER BV, NL, vol. 1, no. 1-6, 1990, pages 425 - 432, XP027419744, ISSN: 0925-4005, [retrieved on 19900101]
- WEHRSPOHN R B ET AL: "ELECTROCHEMICALLY-PREPARED 2D AND 3D PHOTONIC CRYSTALS", PHOTO CRYSTALS: ADVANCES IN DESIGN, FABRICATION ANDCHARACTERIZATION, WILEY-VCH VERLAG, DE, 6 May 2005 (2005-05-06), XP002410630

## Description

La présente invention concerne la réalisation d'une structure poreuse à fort élancement pour la fabrication d'une membrane poreuse pour le transfert d'un fluide et apte à transmettre un rayonnement infrarouge, notamment en vue de mesurer une grandeur physique dans l'infrarouge caractéristique d'un objet ou d'un organisme vivant.

Dans le domaine des sciences du vivant et des sciences de l'environnement, il est connu de caractériser des modes de vibration ou de mesurer des spectres d'absorption dans l'infrarouge pour, par exemple, discriminer des tissus sains de tissus pathologiques, localiser des tumeurs sur des prélèvements anatomo-pathologiques, identifier des éléments pathogènes dans un tissu, ou pour doser des composés chimiques indésirables tels que des pesticides ou des perturbateurs endocriniens.

Il est par exemple connu de FR 3 108 983 A1, de caractériser des colonies de microorganismes cultivés sur une membrane en oxyde d'aluminium, au moyen d'un rayonnement infrarouge et en transmission à travers la membrane. Une telle membrane est poreuse et transfère le milieu nutritif aux microorganismes dans les pores qui la traversent dans son épaisseur. Cependant, L'oxyde d'aluminium est complètement opaque aux longueurs d'onde dans l'infrarouge supérieures à 10 µm, ce qui limite la quantité d'informations qui peuvent être acquises pour caractériser la colonie.

L'invention vise donc à proposer une nouvelle membrane pour transférer un fluide, par exemple l'eau, un alcool ou un milieu nutritif pour la culture de microorganismes, et qui présente une transmittance dans l'infrarouge sur une plage de longueurs d'onde plus étendue qu'une membrane en alumine ainsi qu'un procédé de fabrication d'une telle membrane.

L'invention concerne une membrane de transfert fluidique comportant une paroi poreuse en silicium dopé n comportant des pores s'étendant de part en part dans son épaisseur, chaque pore ayant un diamètre supérieur ou égal à 50 nm et inférieur ou égal à 400 nm et un rapport d'élancement supérieur ou égal à 20.

La membrane transmet ainsi un rayonnement sur une plage de longueurs d'onde plus étendue qu'une membrane en alumine. De préférence, la membrane présente une transmittance supérieure à 5 % à tout rayonnement dont la longueur d'onde est comprise entre 5 µm et 25 µm.

De préférence, la membrane présente une transmittance supérieure ou égale à 10 %, voire supérieure ou égale à 20 %, mieux supérieure ou égale à 30 % à tout rayonnement dont la longueur d'onde est comprise entre 5 µm et 25 µm.

La membrane peut présenter une transmittance supérieure ou égale à 20 % à tout rayonnement dont la longueur d'onde est comprise entre 10 µm et 25 µm.

De préférence, chaque pore présente un diamètre inférieur ou égal à 350 nm.

De préférence, les pores présentent un rapport d'élancement supérieur ou égal à 50, mieux supérieur ou égal à 100.

De préférence, les pores s'étendent transversalement entre les faces de la paroi sur lesquelles ils débouchent, *i.e.* sensiblement perpendiculairement auxdites faces.

La forme des pores n'est pas limitative. Les pores peuvent présenter une forme variée, notamment tubulaire, par exemple cylindrique de révolution.

Les pores peuvent présenter un diamètre variant le long de leur axe d'extension entre les ouvertures par lesquelles ils débouchent, de préférence de 100 % au plus en valeur absolue. Notamment, les pores peuvent présenter, entre les ouvertures par lesquelles ils débouchent, des faces latérales qui ondulent le long de leur axe d'extension.

De préférence, la longueur de chaque pore est supérieure ou égale à 10 µm, de préférence supérieure ou égale à 30 µm, mieux supérieure ou égale à 50 µm.

Les ouvertures des pores peuvent être de forme variée. En particulier, elles peuvent être de forme convexe, notamment polygonale, par exemple carrée, elliptique, circulaire ou en croix.

De préférence, la densité surfacique de pores est comprise entre 0,1 µm⁻² et 5,0 µm⁻².

La paroi poreuse peut présenter une épaisseur supérieure ou égale à 10 µm, de préférence supérieure ou égale à 30 µm, mieux supérieure ou égale à 50 µm.

La paroi poreuse est dopée n, par exemple au phosphore.

De préférence, la membrane présente une forme générale d'une plaque, de préférence plane s'étendant entre deux grandes faces.

L'angle formé par les grandes faces opposées de la membrane est inférieur ou égal à 5°. En particulier, les grandes faces opposées de la membrane peuvent être parallèles.

De préférence, pour améliorer la transmittance de la membrane, au moins une, de préférence chacune des faces de la membrane présente une rugosité de surface inférieure à 20 nm. La rugosité de surface peut être mesurée par microscopie à force atomique.

De préférence, au moins une partie, de préférence l'intégralité de l'aire d'au moins une, de préférence de chacune des grandes faces opposées de la membrane, est exempte d'un revêtement la recouvrant. Un tel revêtement a pour effet de réduire la transmittance de la membrane.

La membrane peut présenter une longueur comprise entre 20 µm et 90 mm. Par exemple, elle est adaptée aux caractéristiques d'un dispositif d'analyse infrarouge, par exemple à la taille du faisceau, sur lequel elle est montée.

La membrane peut consister en la paroi poreuse. La membrane comporte en outre un support superposé à la paroi poreuse et qui comporte au moins un évidement traversant le support de part et part dans son épaisseur, au moins un pore débouchant dans l'évidement. Le support confère ainsi une rigidité et une résistance mécanique à la membrane qui facilite sa préhension et sa manipulation. La longueur de l'évidement, mesurée dans le plan médian de la membrane, est supérieure à 1 mm. Elle peut être supérieure à 3 mm, voire supérieure à 5 mm. Ainsi, l'évidement est suffisamment long pour que son interaction avec un rayonnement n'induise pas ou n'induise qu'une faible réduction de la transmittance de la membrane.

De préférence, le support et la paroi poreuse s'étendent dans des plans parallèles. L'épaisseur du support peut être comprise entre 100 µm et 3 mm, notamment inférieure ou égale à 1 mm.

Le support peut comporter, voire être constitué par, du silicium. Il peut être fait du même matériau que la paroi poreuse.

Le support peut être au contact de la paroi poreuse.

Le support peut être collé sur la paroi poreuse. Il peut être collé par une technique de collage direct de type hydrophobe, notamment par collage silicium sur silicium. En variante, il est collé par une technique de collage de surfaces hydrophiles mettant en œuvre par exemple, un oxyde de silicium, Les surfaces peuvent être déposées, préparées par des activations plasma azote ou oxygène ou par un procédé de polissage mécano-chimique. Le collage peut impliquer d'autres types de matériaux tels que des matériaux organiques d'origine polymère, ou métalliques.

De préférence, le support est dense, *i.e.* qu'il présente une porosité totale inférieure à 0,1 %, la porosité totale du support étant égale au rapport du volume total des pores du support divisé par le volume du support.

Le support et la paroi peuvent former un ensemble monolithique.

L'invention concerne aussi un procédé de fabrication de la membrane selon l'invention, le procédé comportant :
- La fourniture d'un substrat de silicium dopé n qui présente une résistivité électrique comprise entre 1 Ω.cm et 10 Ω.cm, et d'une cellule électrochimique comportant un générateur électrique, une cathode et une anode alimentées électriquement par le générateur électrique,
   la cellule électrochimique contenant un électrolyte aqueux comportant de l'acide fluorhydrique dans une concentration massique supérieure ou égale à 10 %,
   le substrat étant disposé entre l'anode et la cathode,
   au moins une des faces du substrat de silicium étant au contact de l'électrolyte,
   la cathode et la face du substrat de silicium en regard de la cathode, dite « face avant », étant au contact de l'électrolyte, la face avant du substrat de silicium étant distante de la cathode,
- la gravure électrochimique du substrat en appliquant avec le générateur électrique une tension électrique constante et supérieure ou égale à 10 V entre l'anode et la cathode, afin de former des pores borgnes dans le substrat qui présentent chacun un diamètre supérieur ou égal à 50 nm et inférieur ou égal à 400 nm qui s'étendent depuis une face du substrat sur une profondeur supérieure ou égale à 10 µm, la face arrière du substrat étant illuminée pendant la gravure électrochimique avec un rayonnement lumineux afin de générer des porteurs de charge dans le substrat, et
- l'ablation d'une partie du substrat pour que lesdits pores soient débouchants et traversent au moins une portion du substrat de part en part dans son épaisseur.

Le procédé selon l'invention permet ainsi de fabriquer une membrane dans laquelle les pores ont un diamètre inférieur ou égal à 400nm, de préférence compris entre 50 nm et 350 nm. De tels pores, présentant une taille plus de dix fois inférieure aux longueurs d'ondes du rayonnement dans l'infrarouge comprises entre 5 µm et 25 µm, ne diffusent et/ou ne diffractent substantiellement pas ce rayonnement qui peut ainsi être facilement transmis à travers la membrane. En outre, la mise en œuvre du procédé selon l'invention permet de produire rapidement la membrane avec une vitesse de gravure des pores de préférence supérieure à 4 µm.min⁻¹.

De préférence, le substrat présente une résistivité électrique comprise 3 Ω.cm et 6 Ω.cm.

L'électrolyte aqueux est liquide. De préférence, il présente une concentration massique en acide fluorhydrique supérieure ou égale à 15 %, de préférence supérieure ou égale à 25 %, de préférence supérieure ou égale à 30 %, mieux supérieure ou égale à 35 %. La concentration massique en acide fluorhydrique de l'électrolyte aqueux peut être inférieure ou égale à 50 %.

L'électrolyte aqueux peut être obtenu par dilution dans un solvant aqueux, notamment dans l'eau, d'au moins un composé choisi parmi le fluorure d'hydrogène, le fluorure d'ammonium et leurs mélanges.

L'électrolyte aqueux peut en outre contenir un additif, tel que l'isopropanol et/ou l'acide acétique.

L'anode et/ou la cathode peuvent être métalliques, par exemple en platine. En variante, L'anode et/ou la cathode peuvent être en silicium.

Le procédé peut comporter préalablement à la gravure électrochimique, l'illumination du substrat avec un rayonnement lumineux afin de générer des porteurs de charge dans le substrat.

Préalablement à la gravure électrochimique, le substrat peut présenter une épaisseur comprise entre 100 µm et 3 mm, par exemple comprise entre 500 µm et 1,5 mm.

Le substrat peut présenter une orientation (100) ou (111) parallèle à une normale à une de ses faces. Il présente de préférence une forme d'une plaque plane.

De préférence, la cathode présente une face sensiblement plane qui est entièrement superposée à la face avant du substrat. De préférence, la face de la cathode en regard de la face avant du substrat et ladite face avant sont parallèles.

Selon une première variante, l'anode est à distance de la « face arrière » du substrat, qui est opposée la face avant du substrat.

De préférence, l'anode est non superposée à la « face arrière » du substrat.

La cellule électrolytique peut définir des première et deuxième chambres étanches qui sont obturées par le substrat, la première chambre contenant l'électrolyte, la deuxième chambre comportant l'électrolyte ou un autre électrolyte aqueux. La face avant et la face arrière du substrat définissent respectivement une paroi de la première chambre et une paroi de la deuxième chambre. Les première et deuxième chambres étant étanches, les liquides contenus dans ces chambres ne se mélangent pas.

Par ailleurs, afin d'illuminer la face arrière du substrat, la cellule électrochimique peut comporter une fenêtre transparente au rayonnement d'illumination, qui délimite la deuxième chambre.

Le substrat peut être entièrement immergé dans l'électrolyte. La face arrière du substrat, opposée à la face avant, est alors au contact de l'électrolyte. Chacune des première et deuxième chambres contient l'électrolyte. En alternative, la deuxième chambre peut comporter un autre électrolyte aqueux différent de l'électrolyte contenu dans la première chambre. Cet autre électrolyte est par exemple une solution aqueuse de sulfate de potassium K₂SO₄.

Selon une deuxième variante, l'anode est au contact de la face arrière du substrat et recouvre partiellement la face arrière du substrat.

L'illumination du substrat est de préférence effectuée avec un rayonnement lumineux ayant une énergie supérieure à 1.1 eV. Par exemple, elle est produite par une ou plusieurs lampes halogènes d'une puissance lumineuse supérieure ou égale à 500 lumens.

La gravure électrochimique est effectuée à tension électrique constante, c'est-à-dire en mode potentiostatique. De préférence, elle est effectuée à une tension électrique constante comprise entre 25 V et 100 V.

De préférence, la gravure électrochimique est conduite jusqu'à ce que les pores s'étendent chacun, à partir d'une face du substrat sur une profondeur supérieure ou égale à 30 µm, de préférence supérieure ou égale à 50 µm, voire supérieure ou égale à 75 µm.

De préférence, la partie du substrat qui est ablatée s'étend de la face du substrat opposée à la face sur laquelle les pores borgnes débouchent jusqu' au fond des pores borgnes.

Préalablement à l'étape d'ablation, le procédé peut comporter un polissage mécanochimique de la face du substrat sur laquelle les pores borgnes débouchent, afin d'abraser une couche surfacique de silicium nanoporeux ayant une épaisseur comprise entre 50 nm et 1 µm, formée sur ladite face lors de la gravure électrochimique.

Par ailleurs, l'ablation peut être opérée par polissage mécanique, gravure chimique par voie humide et/ou gravure profonde par voie sèche.

De préférence, l'ablation est sélective afin d'extraire une partie seulement de la portion du substrat superposée aux pores borgnes, de préférence pour former au moins un évidement par lequel au moins un, de préférence plusieurs des pores débouchent.

De préférence, l'ablation sélective comporte la gravure profonde du substrat. La gravure profonde comporte l'application d'un masque de protection, de préférence par photolithographie d'une résine photosensible, sur la face du substrat opposée à la face par laquelle les pores borgnes débouchent. Le masque de protection comporte au moins une fenêtre le traversant de part en part dans son épaisseur. La gravure profonde peut être chimique et comporter la mise en contact de la face du substrat recouverte par le masque de protection avec un agent chimique d'ablation, par exemple une composition aqueuse à base d'acide fluorhydrique, de manière à extraire de la matière d'une zone du substrat superposée à la ou aux fenêtres. En variante, la gravure profonde peut être physique et comporter l'ablation de la matière dans une zone du substrat superposée à la ou aux fenêtres par bombardement de la face du substrat recouverte par le masque de protection au moyen d'un plasma. Le plasma peut comporter un gaz réactif ionisé, notamment fluoré, par exemple choisi parmi SF₆, CF₄ et leurs mélanges.

De préférence, préalablement à l'étape d'ablation, le procédé peut comporter le dépôt d'une couche de protection, de préférence formée d'au moins un oxyde, notamment l'oxyde de silicium, par exemple déposé par dépôt chimique en phase vapeur (CVD) à partir de tétraéthylorthosilicate (TEOS ou de SiH₄ (silane)), sur la face du substrat sur laquelle le support débouche. Le procédé peut en outre comporter la suppression de la couche de protection postérieurement à l'étape d'ablation.

Le procédé peut comporter un traitement ultérieur de la membrane, par exemple un traitement de fonctionnalisation, pour rendre la membrane hydrophile ou hydrophobe, afin de faciliter la diffusion d'espèces chimiques spécifiques.

L'invention concerne également une membrane obtenue par le procédé selon l'invention.

L'invention concerne enfin une méthode de caractérisation d'une colonie de microorganismes, comportant :
- la culture des microorganismes dans un milieu nutritif, les microorganismes étant disposés sur une face d'une membrane selon l'invention et/ou obtenue par le procédé selon l'invention, la membrane étant au contact du milieu nutritif de telle sorte que le milieu nutritif est transféré par les pores aux microorganismes,
- l'illumination des microorganismes en transmission à travers la membrane par un rayonnement lumineux ayant au moins une longueur d'onde comprise entre 5 µm et 25 µm et l'acquisition du rayonnement lumineux ayant interagi avec les microorganismes.

La « transmittance » T d'un corps d'un rayonnement incident de longueur d'onde λ est égale au rapport de l'intensité I du rayonnement transmis de longueur d'onde λ sur l'intensité I₀ du rayonnement incident, *i.e.* : [Math. 1] $T = \frac{I}{{I}_{0}}$

La transmittance T peut être mesurée par spectrométrie infrarouge.

La transmittance peut être liée à plusieurs caractéristiques structurales de la membrane, par exemple la structure poreuse, la rugosité des surfaces de la membrane, la largeur et/ou la longueur de reliefs en saillie ou en creux formés en surface de la membrane.

Le « rapport d'élancement » d'un pore est égal au rapport de la longueur du pore sur le diamètre du pore.

Le « diamètre » d'un pore est égal à la moyenne des diamètres des ouvertures du pores sur les deux faces respectives opposées de la paroi sur lesquelles le pore débouche, ou si le pore est borgne, le diamètre du pore est égal au diamètre de l'ouverture par lequel il débouche. Le diamètre de l'ouverture d'un pore est égal au diamètre du plus petit cercle circonscrit à l'ouverture dudit pore.

La « longueur » d'un pore est la distance entre les ouvertures par lesquelles le pore débouche sur les faces opposées de la paroi, ou si le pore est borgne, la distance entre l'ouverture par lequel le pore débouche et le fond du pore.

La « densité surfacique » de pores est déterminée sur une face de la paroi ou du substrat le cas échéant. Elle est égale au rapport de l'aire totale occupée par les ouvertures des pores sur une zone de la face de la paroi ou du substrat respectivement, sur l'aire de ladite zone. De préférence, l'aire de ladite zone sur laquelle la densité surfacique de pores est déterminée est comprise entre 2 µm² et 500 µm².

La « résistivité » du substrat est mesurée par la méthode des quatre pointes.

La « concentration massique » en acide fluorhydrique de l'électrolyte aqueux correspond au rapport de la masse d'acide fluorhydrique dans l'électrolyte aqueux sur la masse de l'électrolyte aqueux.

La « vitesse de gravure » des pores est égale au rapport de la longueur d'un pore sur la durée de l'étape de gravure.

L' « anode » est une électrode où se produit une réaction d'oxydation au sens électrochimique, *i.e.* un transfert électronique de l'électrolyte vers l'électrode, ou transfert de trous de l'électrode vers l'électrolyte.

La « cathode » est une électrode où se produit une réaction de réduction au sens électrochimique, *i.e.* un transfert électronique de l'électrode vers l'électrolyte.

L'invention pourra être mieux comprise à la lecture de la description détaillée et du dessin qui va suivre dans lequel :
- [Fig. 1] représente un exemple de cellule électrochimique pour la mise en œuvre du procédé selon l'invention,
- [Fig. 2], [Fig. 3a], [Fig.3b], [Fig.4], [Fig.5], [Fig.6], [Fig.7], [Fig.8] et [Fig. 9] sont des photographies acquises en microscopie électronique à balayage des substrats gravés des exemples 1 à 4 et 6 à 9 respectivement,
- [Fig. 10] représente l'évolution de la transmittance de différents substrats en fonction de la longueur d'onde,
- [Fig. 11] et [Fig. 12] sont des photographies acquises en microscopie électronique à balayage des substrats gravés des exemples 10 et 11 respectivement,
- [Fig. 13] représente l'évolution de la transmittance de substrats des exemples 10 et 11 en fonction de la longueur d'onde,
- [Fig. 14] représente schématiquement les étapes de réalisation d'une membrane selon un exemple de mise en œuvre du procédé, et
- [Fig. 15] illustre un autre exemple de cellule électrochimique pour la mise en œuvre du procédé selon l'invention.

La littérature scientifique est abondante en ce qui concerne la réalisation de membranes de silicium pour de multiples applications, comme cela est par exemple synthétisé dans l'article de revue « Porous silicon membranes and their applications : Recent advances », R. Vercauteren et al., Sensors and Acuators, A318 (2021), 112486. Par exemple, les articles
- « Thick macroporous membranes made of p-type silicon », J. Zheng et al., Phys. Status, Solidi. A, vol. 202, num. 8, pages 1402-1406 (2005),
- « Integration of low-loss inductors on thin porous silicon membranes », B. Bardet et al., Microelectron. Eng., vol. 194, pages 96-99 (2018),
- « Highly insulating, fully porous silicon substrates for high temperature micro-hotplates », F. Lucklum et al., Sensor. Actuat. A Phys., vol. 213, pages 35-42 (2014),
- « All-in-One Nanowire-Decorated Multifunctional Membrane for RapidCell Lysis and Direct DNA Isolation », H. So et al., ACS Appl. Mater Interfaces, vol 6, pages 20693-20699 (2014),
- « Electrochemically etched nanoporous silicon membrane for separation of biological molecules in mixture », N. Burham et al., J. Micromech. MicroEng., vol. 27, page 075021 (2017), et
- « Effects of nano/microstructures on performance of Si-based microfuel cells », Appl. Therm. Eng., Vol. 72, num. 2, Pages 298-303 (2014)
décrivent différents procédés de fabrication de membranes de silicium.

Le procédé selon l'invention met en œuvre une gravure électrochimique d'un substrat dans un électrolyte aqueux à base d'acide fluorhydrique. D'une manière générale, la gravure électrochimique du silicium est une technique connue et documentée de longue date, comme par exemple dans le livre « Electrochemistry of Silicon: Instrumentation, Science, Materials and Applications », Dr. Volker Lehmann, doi : 10.1002/3527600272, 2002 Wiley-VCH Verlag GmbH.

Les conditions de mise en œuvre du procédé selon l'invention sont toutefois non conventionnelles. A la connaissance des inventeurs, jusqu'à l'invention, les travaux sur la gravure électrochimique du silicium avaient principalement été orientés vers la recherche de pores ayant un profil et des parois réguliers. Dans ce cadre, les études, pour ces caractéristiques de substrat, étaient principalement focalisées sur des pores de diamètre supérieur à 1 µm et jusqu'à environ 10 µm et présentant des formes tubulaires les plus régulières possibles et exemptes de défaut sans qu'un intérêt ne soit porté aux propriétés de transmission dans l'infrarouge. A cette fin, des gravures de substrats de silicium avaient été effectuées dans des conditions différentes de l'invention, par exemple en mode galvanostatique ou en mode potentiostatique mais à une faible tension électrique inférieure à 10 V, et dans un électrolyte aqueux où la concentration massique en acide fluorhydrique était faible, *i.e.* inférieure à 10 %.

### Exemples

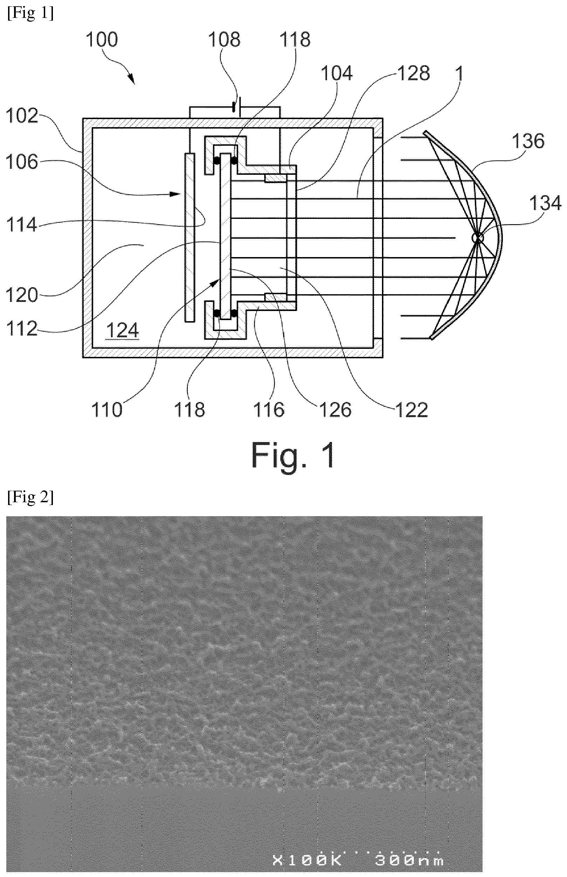

Les exemples 1 et 2 comparatifs et 3 à 9 selon l'invention ont été réalisés au moyen de la cellule électrochimique illustrée sur la figure 1.

La cellule électrochimique 100 comporte une enceinte 102, une anode 104 et une cathode 106 en silicium étant disposées dans l'enceinte et reliées électriquement à un générateur électrique 108. Un substrat 110 de silicium a été logé entre l'anode et la cathode. La face avant 112 du substrat est en regard d'une face 114 de la cathode.

L'enceinte comporte en outre un boitier 116 dont les parois sont pleines, le substrat de silicium obturant le boitier. Des joints d'étanchéité 118 sont disposés de part et d'autre des extrémités du substrat. Ainsi, des première 120 et deuxième 122 chambres sont définies qui sont étanches l'une de l'autre. Un électrolyte aqueux 124 comportant de l'acide fluorhydrique dont la concentration massique a été modifiée pour les différent essais, a été introduit dans chacune des deux chambres. Ainsi, les faces avant 110 et arrière 126 du substrat ont été au contact de l'électrolyte, si bien qu'elles ont agi comme des électrodes pour assurer la continuité électrique lors des réactions d'oxydo-réduction.

Par ailleurs, la partie 128 de la paroi du boitier superposée à la face arrière 126 est transparente à la lumière, et une illumination I a été générée par des lampes à halogène 134 et répartie sur l'ensemble de la face arrière du substrat au moyen d'un miroir réfléchissant 136 de forme hémisphérique, afin de générer des porteurs de charges dans le substrat.

Le substrat était une plaque de silicium qui présentait une épaisseur de 750 µm, était en silicium dopé par du phosphore, et donc de type n, et présentait une résistivité électrique comprise entre 3 Ω.cm et 6 Ω.cm.

Pendant la mise en œuvre de la gravure électrochimique, la plaque a été illuminée par un rayonnement lumineux de puissance supérieure à 20000 lumens au moyen de lampes halogènes.

La gravure électrochimique de la face avant du substrat a été effectuée sous un régime potentiostatique, avec une tension maintenue constante entre l'anode et la cathode de la cellule tout au long de la gravure.

La durée de gravure électrochimique a été maintenue constante à 6 minutes pour tous les essais, excepté pour les essais 2 et 6 pour lesquels la durée était de 15 min.

Le tableau 1 ci-dessous synthétise les conditions expérimentales et les résultats obtenus. Les essais marqués d'un astérisque sont comparatifs. « N.A. » signifie « non applicable »

**[Tableau 1]**

| Essai | Tension (V) | Concentration massique d'acide fluorhydrique (%) | Densité surfacique de pores (µm⁻²) | Diamètre des pores (nm) | Vitesse de gravure des pores (µm/min) | Longueur des pores (µm) |
|---|---|---|---|---|---|---|
| 1^{(*)} | 5 | 15 | N.A | N.A. | N.A. | N.A. |
| 2^{(*)} | 15 | 5 | 0,05 | 1600 | 0,8 | ~ 15 µm |
| 3 | 15 | 15 | 0,20 | 230 | 5,3 | ~ 32 µm |
| 4 | 15 | 35 | 0,38 | 320 | 10,1 | ~ 50 µm |
| 5 | 25 | 15 | 0,45 | 230 | 5,8 | ~ 35 µm |
| 6^{(*)} | 35 | 5 | 0,35 | 420 | 1,1 | ~ 15 µm |
| 7 | 35 | 15 | 0,65 | 220 | 6,5 | ~ 40 µm |
| 8 | 35 | 35 | 0,50 | 150 | 9,7 | ~ 60 µm |
| 9 | 45 | 35 | 0,85 | 300 | 11,2 | ~ 65 µm |

Dans le cas de l'exemple 1, hors invention, l'application d'une faible tension de 5 V, telle qu'appliquée conventionnellement jusqu'à l'invention, avec une concentration massique en acide fluorhydrique de 15 %, ne permet pas d'effectuer une gravure du substrat. Cela est observé sur la figure 2, la face observée du substrat a été attaquée chimiquement. Le résultat est une surface rugueuse recouverte d'agrégats de silicium homogènement répartis en surface, mais aucun pore n'a été formé.

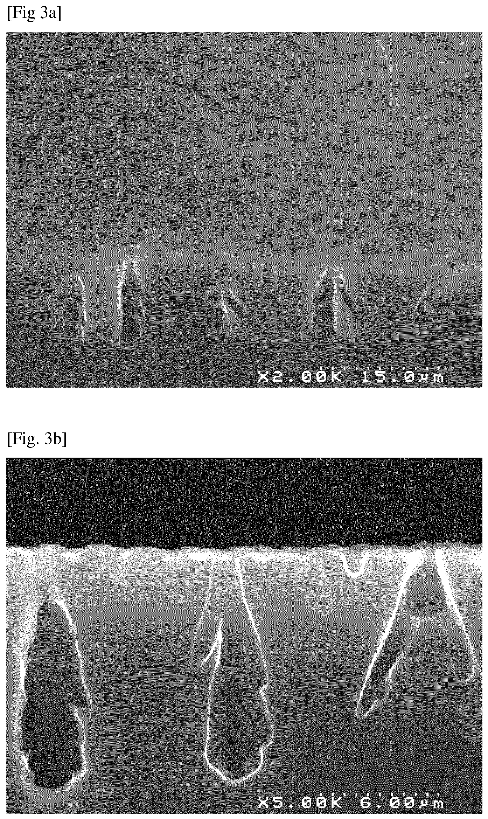

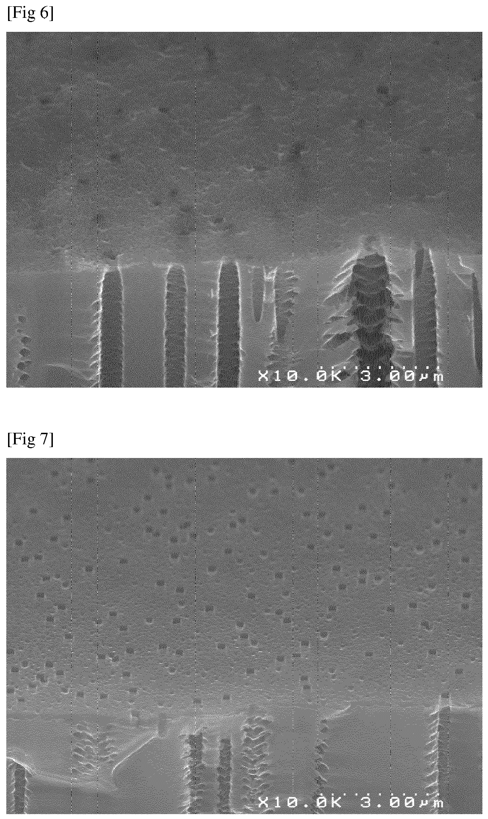

Dans le cas de l'exemple 2, hors invention, l'application d'une tension de 15 V, non conventionnelle jusqu'à l'invention avec une concentration massique en acide fluorhydrique de 5 %, résulte en une lente formation de pores d'un diamètre de 1600 nm environ, observable sur la figure 3a et sur l'agrandissement de la figure 3b. Une augmentation de la tension à 35 V, comme selon l'exemple 6 hors invention ne permet pas d'atteindre une taille de pores de 400 nm au plus comme selon l'invention, observable sur la figure 6. En outre, dans ces exemples comparatifs, la vitesse de gravure est trop lente, inférieure à 2 µm/min.

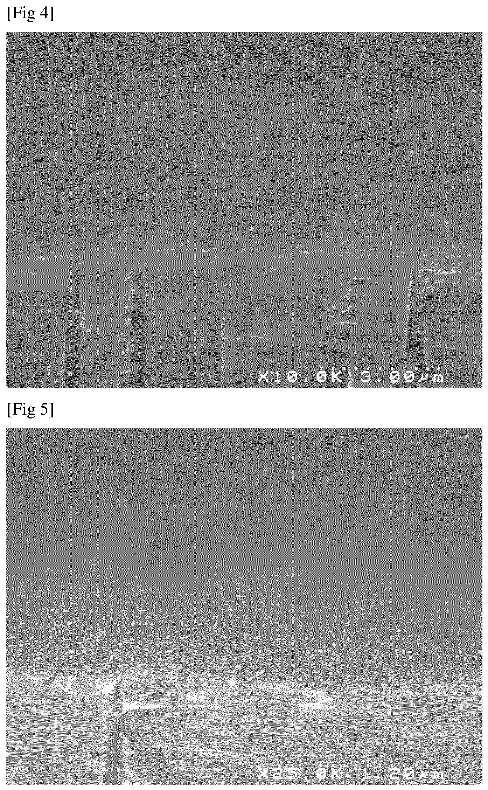

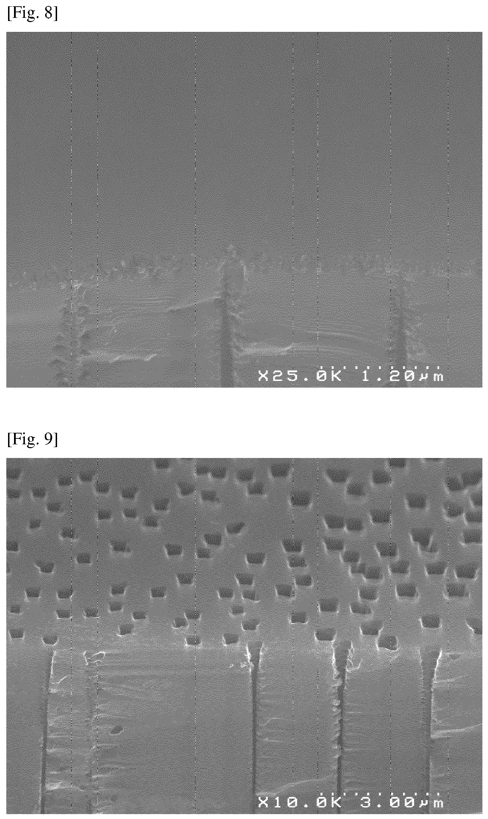

Comme cela peut être observé sur les figures 4, 5 et 7 à 9, correspondant aux exemples 3, 4 et 7 à 9, la mise en œuvre du procédé selon l'invention permet de fabriquer un substrat dans lesquels les pores présentent un diamètre compris entre 50 nm et 400 nm.

Dans les exemples illustrés ci-dessus, en fin de gravure électrochimique, les pores obtenus étaient borgnes et débouchaient par une unique ouverture sur une face du substrat.

Des mesures de transmittance ont été effectuées sur les substrats ainsi gravés.

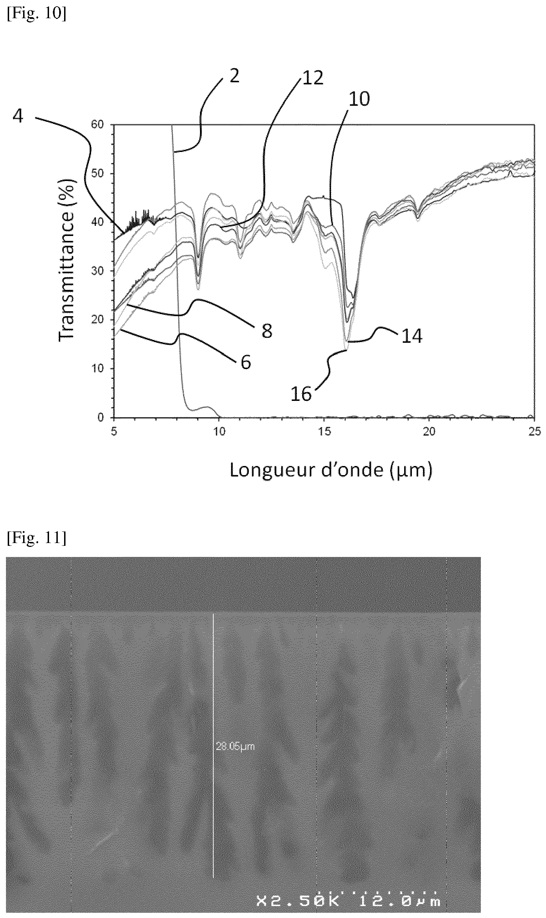

La transmittance des substrats gravés objets des exemples 3 à 5 et 7 à 9, a été mesurée sur le spectre de longueurs d'onde dans l'infrarouge comprise entre 5 µm et 25 µm et est représentée sur la figure 10.

La courbe 2 représente l'évolution de la transmittance d'une couche d'alumine poreuse comportant des pores cylindriques traversant d'un diamètre compris en 20 nm et 200 nm, d'une épaisseur de 60 µm et répartis selon une densité surfacique variant entre 10 et 2000 µm⁻².

Comme cela apparaît sur la figure 10, cette couche d'alumine présente une transmittance élevée jusqu'à une longueur d'onde de 10 µm mais est totalement opaque aux rayonnement de longueurs d'ondes supérieures.

L'évolution de la transmittance pour un substrat dense de type n et de résistivité .de 3-6 Ω.cm est illustrée par la courbe 4. Ce substrat présente une transmittance supérieure à 20 % sur toute la plage de longueurs d'onde entre 5 µm et 25 µm. Cependant, étant dépourvu de pores, il ne peut former une membrane pour le transfert fluidique.

Les courbes 6 à 16 illustrent l'évolution de la transmittance pour les exemples 3, 4, 5, 7, 8 et 9 selon l'invention respectivement. Comme cela est observé sur la figure 10, les substrats gravés de ces exemples présentent donc une transmittance proche de celle du substrat dense. Ceci confirme que les pores d'un diamètre et d'un élancement selon l'invention n'interagissent que faiblement avec le rayonnement infrarouge, et permettent de conserver les bonnes propriétés de transmission à l'infrarouge dans la plage de longueurs d'onde de 5 µm à 25 µm du substrat de silicium dopé n.

A titre comparatif, des substrats de type n identiques à ceux des exemples 1 à 9 ont été soumis à une étape de gravure électrochimique dans la même cellule électrochimique, mais en condition galvanostatique, c'est-à-dire en imposant une densité de courant constante. Ainsi, l'exemple 10 a été produit, avec le même dispositif expérimental, en immergeant l'anode dans la solution d'acide fluorhydrique à une concentration de 35 % puis en effectuant la gravure électrochimique sous une densité de courant constante de 14 mA.cm⁻². L'exemple 11 a été réalisé dans des conditions identiques à celles de l'exemple 10, à ceci près que la concentration en acide fluorhydrique était de 15 % et que la densité de courant constante imposée était de 20 mA.cm⁻².

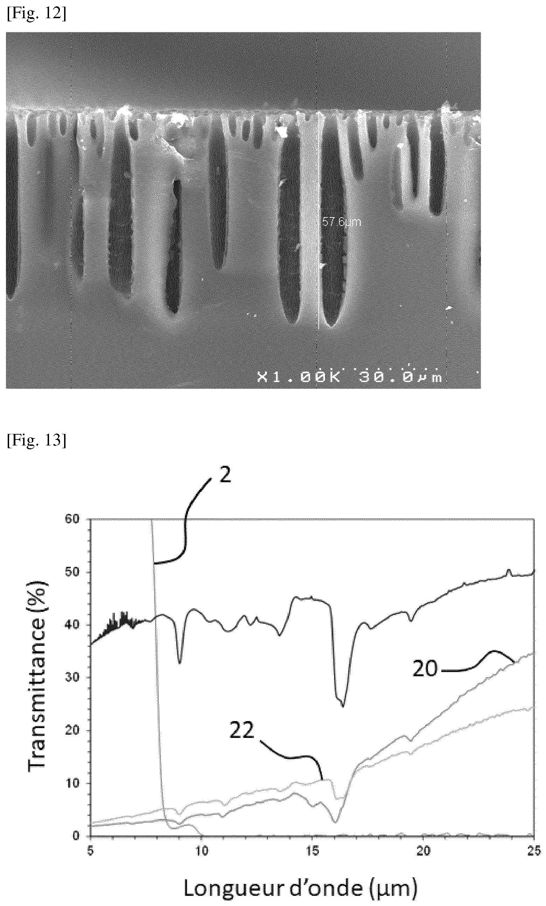

Comme cela est observé sur les figures 11 et 12, des pores borgnes ont été formés mais qui présentaient tous des pores d'un diamètre supérieur à 2 µm. Ces pores de grande taille interagissent avec le rayonnement infrarouge et réduisent la transmittance du substrat de type n. Ceci est observé sur la figure 13. Les courbes 20 et 22 représentent l'évolution de la transmittance des exemples 10 et 11 respectivement. La transmittance de ces substrats gravés, hors invention, est inférieure à 5 % pour les rayonnements de longueur d'onde comprise entre 5 µm et 8 µm. Elle est inférieure à 10 % pour les rayonnements de longueur d'onde inférieure à 17 µm pour l'exemple 10 et inférieure à 14 µm pour l'exemple 11.

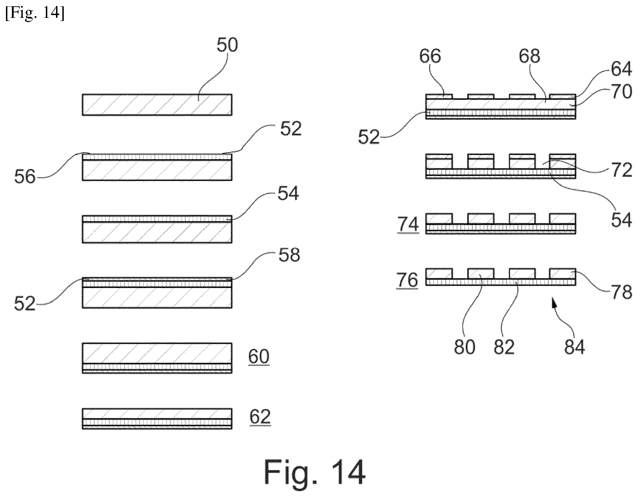

Par ailleurs, une membrane a été formée pour une paroi poreuse obtenue selon les conditions de l'exemple 3, comme illustré sur la figure 14.

La face 52 du substrat 50 sur laquelle débouchaient les pores 54 borgnes a été polie afin de supprimer une couche 56 de silicium nanoporeux formée en surface par la gravure électrochimique. Ensuite, une couche de protection 58 faite d'oxyde de silicium (de type TEOS ou SiH₄ déposé par CVD) a été déposée sur la face 52 polie pour éviter que les ouvertures des pores ne soient endommagées par l'ablation ultérieure. Le substrat a été retourné (étape 60) puis abrasé mécaniquement (étape 62) pour réduire l'épaisseur du substrat. Une couche de résine photosensible 64 a ensuite été déposée sur la face 66 du substrat opposé à la face 52, et a été ablatée localement sous l'effet d'un rayonnement lumineux dans l'ultraviolet selon les procédés classiques de photolithographie. Des zones 68 de la portion 70 du substrat superposé aux pores borgnes ont été ablatées par gravure profonde par plasma, de manière à former des évidements 72 dans le substrat, délimités par un masque, jusqu'à ce que les pores 54 débouchent dans l'évidement. La résine photosensible et la couche d'oxyde ont ensuite été supprimées (étape 74 et 76).

Ainsi, les zones non ablatées 78 du substrat forment un support 80 pourvu d'évidements, auquel une paroi 82 est superposée, pourvue de pores borgnes débouchant sur les zones non ablatées et de pores traversant la paroi de part en part dans son épaisseur et débouchant par une de leurs ouvertures dans un des évidements. Une membrane 84 est ainsi obtenue.

L'invention n'est pas limitée aux exemples et modes de réalisations et de mise en œuvre optionnels présentés à titre illustratif et non limitatif.

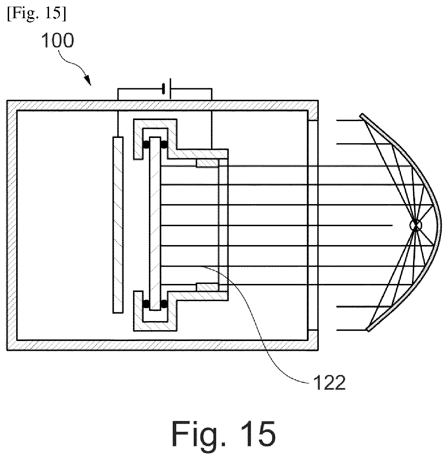

Notamment, le procédé peut être mis en œuvre au moyen de la cellule électrochimique illustrée sur la figure 15. La cellule 100 illustrée sur la figure 15 diffère de celle illustrée sur la figure 1 en ce que la deuxième chambre 122 contient un autre électrolyte, par exemple à base de sulfate de potassium, différent de celui contenu dans la première chambre.

## Revendications

1. Membrane (84) de transfert fluidique comportant une paroi (82) poreuse en silicium comportant des pores (54) s'étendant de part en part dans son épaisseur, chaque pore ayant un rapport d'élancement supérieur ou égal à 20, la membrane présentant une transmittance supérieure ou égale à 5 % à tout rayonnement dont la longueur d'onde est comprise entre 5 µm et 25 µm, la membrane comportant en outre
un support (80) superposé à la paroi poreuse et qui comporte au moins un évidement (72) traversant le support de part et part dans son épaisseur, au moins un pore (54) débouchant dans l'évidement (72), la longueur de l'évidement (72), mesurée dans le plan médian de la membrane, étant supérieure à 1 mm,
la paroi poreuse étant en silicium dopé n, et
chaque pore ayant un diamètre supérieur ou égal à 50 nm et inférieur ou égal à 400 nm.

2. Membrane selon la revendication 1, présentant une transmittance supérieure ou égale à 10 %, voire supérieure ou égale à 20 %, mieux supérieure ou égale à 30 % à tout rayonnement dont la longueur d'onde est comprise entre 5 µm et 25 µm.

3. Membrane selon l'une quelconque des revendications 1 et 2, chaque pore présentant un diamètre inférieur ou égal à 350 nm.

4. Membrane selon l'une quelconque des revendications précédentes, les pores présentent un rapport d'élancement supérieur ou égal à 50, mieux supérieur ou égal à 100.

5. Membrane selon l'une quelconque des revendications précédentes, la densité surfacique de pores étant comprise entre 0,1 µm⁻² et 5,0 µm⁻².

6. Membrane selon l'une quelconque des revendications précédentes, l'angle formé par les grandes faces opposées de la membrane est inférieur ou égal à 5°, les grandes faces opposées de la membrane étant notamment parallèles.

7. Membrane selon l'une quelconque des revendications précédentes, au moins une, de préférence chacune des faces de la membrane présentant une rugosité de surface inférieure à 20 nm.

8. Membrane selon l'une quelconque des revendications précédentes, au moins une partie, de préférence l'intégralité de l'aire d'au moins une, de préférence de chacune des grandes faces opposées de la membrane, étant exempte d'un revêtement la recouvrant.

9. Procédé de fabrication d'une membrane selon l'une quelconque des revendications précédentes, le procédé comportant
- la fourniture d'un substrat de silicium dopé n qui présente une résistivité électrique comprise entre 1 Ω.cm et 10 Ω.cm, et d'une cellule électrochimique comportant un générateur électrique, une cathode et une anode alimenté électriquement par le générateur électrique,
la cellule électrochimique contenant un électrolyte aqueux comportant de l'acide fluorhydrique dans une concentration massique supérieure ou égale à 10 %, le substrat étant disposé entre l'anode et la cathode,
au moins une des faces du substrat de silicium étant au contact de l'électrolyte, la cathode et la face du substrat de silicium en regard de la cathode, dite « face avant », étant au contact de l'électrolyte, la face avant du substrat de silicium étant distante de la cathode,
- la gravure électrochimique du substrat en appliquant avec le générateur électrique une tension électrique constante et supérieure ou égale à 10 V entre l'anode et la cathode, afin de former des pores borgnes dans le substrat qui présentent chacun un diamètre supérieur ou égal à 50 nm et inférieur ou égal à 400 nm et qui s'étendent depuis une face du substrat sur une profondeur supérieure ou égale à 10 µm, la face arrière du substrat étant illuminée pendant la gravure électrochimique avec un rayonnement lumineux afin de générer des porteurs de charge dans le substrat, et
- l'ablation d'une partie du substrat pour que lesdits pores soient débouchants et traversent au moins une portion du substrat de part en part dans son épaisseur.

10. Procédé selon la revendication 9, l'électrolyte aqueux présentant une concentration massique en acide fluorhydrique supérieure ou égale à 15 %, de préférence supérieure ou égale à 30 %, mieux supérieure ou égale à 35 %.

11. Procédé selon l'une quelconque des revendications 9 et 10, comportant préalablement à la gravure électrochimique, l'illumination du substrat avec un rayonnement lumineux afin de générer des porteurs de charge dans le substrat.

12. Procédé selon l'une quelconque des revendications 9 à 11, la gravure électrochimique étant conduite jusqu'à ce que les pores s'étendent chacun à partir d'une face du support sur une profondeur supérieure ou égale à 30 µm, de préférence supérieure ou égale à 50 µm, voire supérieure ou égale à 75 µm.

13. Procédé selon l'une quelconque des revendications 9 à 12, la gravure électrochimique étant effectuée à une tension électrique constante comprise entre 25 V et 100 V.

14. Procédé selon l'une quelconque des revendications 9 à 13, le substrat présentant une épaisseur, préalablement à la gravure électrochimique, comprise entre 100 µm et 3 mm, de préférence comprise entre 500 µm et 1,5 mm.

15. Procédé selon l'une quelconque des revendications 9 à 14, l'ablation étant opérée par polissage mécanique, gravure chimique par voie humide et/ou gravure profonde par voie sèche de préférence l'ablation étant sélective.

16. Procédé selon l'une quelconque des revendications 9 à 15, comportant un traitement ultérieur de la membrane pour rendre la membrane hydrophile ou hydrophobe.

17. Méthode de caractérisation d'une colonie de microorganismes, comportant :
- la culture des microorganismes dans un milieu nutritif, les microorganismes étant disposés sur une face d'une membrane selon l'une quelconque des revendications 1 à 8 et/ou obtenue par le procédé selon l'une quelconque des revendications 9 à 16, la membrane étant au contact du milieu nutritif de telle sorte que le milieu nutritif est transféré par les pores aux microorganismes,
- l'illumination des microorganismes en transmission à travers la membrane par un rayonnement lumineux ayant au moins une longueur d'onde comprise entre 5 µm et 25 µm et l'acquisition du rayonnement lumineux ayant interagi avec les microorganismes.

## Patentansprüche

1. Fluidübertragungsmembran (84), umfassend eine poröse Siliziumwand (82) mit Poren (54), die sich von der einen zur anderen Seite durch ihre Dicke erstrecken, wobei jede Pore ein Aspektverhältnis größer oder gleich 20 aufweist, wobei die Membran eine Transmission größer oder gleich 5 % für jedwede Strahlung mit einer Wellenlänge zwischen 5 µm und 25 µm aufweist, wobei die Membran ferner umfasst
einen Träger (80), der der porösen Wand überlagert ist und mindestens eine Aussparung (72) umfasst, die sich von der einen zur anderen Seite ihrer Dicke durch den Träger erstreckt, wobei mindestens eine Pore (54) in die Aussparung (72) mündet, wobei die Länge der Aussparung (72), gemessen in der Mittelebene der Membran, größer als 1 mm ist,
wobei die poröse Wand aus n-dotiertem Silizium gebildet ist, und
jede Pore einen Durchmesser größer oder gleich 50 nm und kleiner oder gleich 400 nm aufweist.

2. Membran nach Anspruch 1, die eine Transmission größer oder gleich 10 %, oder größer oder gleich 20 %, besonders bevorzugt größer oder gleich 30 % für jedwede Strahlung mit einer Wellenlänge zwischen 5 µm und 25 µm aufweist.

3. Membran nach einem der Ansprüche 1 und 2, wobei jede Pore einen Durchmesser kleiner oder gleich 350 nm aufweist.

4. Membran nach einem der vorhergehenden Ansprüche, wobei die Poren ein Aspektverhältnis größer oder gleich 50, bevorzugt größer oder gleich 100 aufweisen.

5. Membran nach einem der vorhergehenden Ansprüche, wobei die Oberflächendichte der Poren zwischen 0,1 µm⁻² und 5,0 µm⁻² beträgt.

6. Membran nach einem der vorhergehenden Ansprüche, wobei der von den gegenüberliegenden Hauptflächen der Membran gebildete Winkel kleiner oder gleich 5° ist, wobei die gegenüberliegenden Hauptflächen der Membran insbesondere parallel sind.

7. Membran nach einem der vorhergehenden Ansprüche, wobei mindestens eine, vorzugsweise jede der Flächen der Membran eine Oberflächenrauhigkeit von weniger als 20 nm aufweist.

8. Membran nach einem der vorhergehenden Ansprüche, wobei mindestens ein Teil, vorzugsweise der gesamte Flächenbereich mindestens einer, vorzugsweise jeder, der gegenüberliegenden Hauptflächen der Membran frei von einer sie überdeckenden Beschichtung ist.

9. Verfahren zur Herstellung einer Membran nach einem der vorhergehenden Ansprüche, wobei das Verfahren umfasst
- Bereitstellung eines n-dotierten Siliziumsubstrats mit einem elektrischen Widerstand zwischen 1 Ω.cm und 10 Ω.cm und einer elektrochemischen Zelle, umfassend einen elektrischen Generator, eine Kathode und eine Anode, die vom elektrischen Generator elektrisch versorgt wird;
wobei die elektrochemische Zelle einen wässrigen Elektrolyten enthält, der Fluorwasserstoffsäure in einer Massenkonzentration größer oder gleich 10 Gew.-% umfasst, wobei das Substrat zwischen der Anode und der Kathode angeordnet ist,
mindestens eine der Seiten des Siliziumsubstrats in Kontakt mit dem Elektrolyten steht,
wobei die Kathode und die der Kathode zugewandte Fläche des Siliziumsubstrats, die als "Vorderseite" bezeichnet wird, mit dem Elektrolyten in Kontakt stehen, wobei die Vorderseite des Siliziumsubstrats von der Kathode beabstandet ist,
- elektrochemisches Ätzen des Substrats durch Anlegen einer konstanten elektrischen Spannung größer oder gleich 10 V zwischen Anode und Kathode mit dem elektrischen Generator, um sackförmige Poren im Substrat zu bilden, die jeweils einen Durchmesser größer gleich 50 nm und kleiner gleich 400 nm aufweisen und sich von einer Seite des Substrats aus bis zu einer Tiefe größer gleich 10 µm erstrecken, wobei die Rückseite des Substrats während des elektrochemischen Ätzens mit Lichtstrahlung beleuchtet wird, um Ladungsträger in dem Substrat zu erzeugen, und
- Abtragen eines Teils des Substrats, so dass die Poren durchgängig werden und durch mindestens einen Teil des Substrats von der einen zur anderen Seite durch seine Dicke hindurchgehen.

10. Verfahren nach Anspruch 9, wobei der wässrige Elektrolyt eine Fluorwasserstoffsäure-Konzentration größer oder gleich 15 Gew.-%, vorzugsweise größer oder gleich 30 Gew.-%, besonders bevorzugt größer oder gleich 35 Gew.-%, aufweist.

11. Verfahren nach einem der Ansprüche 9 und 10, das vor dem elektrochemischen Ätzen das Beleuchten des Substrats mit Lichtstrahlung beinhaltet, um Ladungsträger in dem Substrat zu erzeugen.

12. Verfahren nach einem der Ansprüche 9 bis 11, wobei das elektrochemische Ätzen durchgeführt wird, bis sich die Poren jeweils ausgehend von einer Seite des Trägers bis zu einer Tiefe größer oder gleich 30 µm, vorzugsweise größer oder gleich 50 µm, oder größer oder gleich 75 µm, erstrecken.

13. Verfahren nach einem der Ansprüche 9 bis 12, wobei das elektrochemische Ätzen bei einer konstanten elektrischen Spannung zwischen 25 V und 100 V durchgeführt wird

14. Verfahren nach einem der Ansprüche 9 bis 13, wobei das Substrat vor dem elektrochemischen Ätzen eine Dicke zwischen 100 µm und 3 mm, vorzugsweise zwischen 500 µm und 1,5 mm aufweist.

15. Verfahren nach einem der Ansprüche 9 bis 14, wobei das Abtragen durch mechanisches Polieren, nasschemisches Ätzen und/oder trockenes Tiefätzen durchgeführt wird, wobei das Abtragen vorzugsweise selektiv erfolgt.

16. Verfahren nach einem der Ansprüche 9 bis 15, umfassend eine anschließende Behandlung der Membran, um die Membran hydrophil oder hydrophob zu machen.

17. Verfahren zur Charakterisierung einer Kolonie von Mikroorganismen, umfassend:
- das Kultivieren der Mikroorganismen in einem Nährmedium, wobei die Mikroorganismen auf einer Seite einer Membran nach einem der Ansprüche 1 bis 8 angeordnet sind und/oder durch das Verfahren nach einem der Ansprüche 9 bis 16 erhalten werden, wobei die Membran mit dem Nährmedium in Kontakt steht, so dass das Nährmedium durch die Poren zu den Mikroorganismen übertragen wird,
- die Beleuchtung von Mikroorganismen in Transmission durch die Membran hindurch mittels einer Lichtstrahlung mit mindestens einer Wellenlänge zwischen 5 µm und 25 µm, und die Erfassung von Lichtstrahlung, die mit den Mikroorganismen interagiert hat.

## Claims

1. Fluid transfer membrane (84) comprising a porous silicon wall (82) comprising pores (54) extending entirely across its thickness, each pore having a slenderness ratio of greater than or equal to 20, the membrane having a transmittance of greater than or equal to 5% to any radiation with a wavelength of between 5 µm and 25 µm, the membrane further comprising
a support (80) superposed on the porous wall and which comprises at least one recess (72) passing through the support entirely across its thickness, at least one pore (54) opening into the recess (72), the length of the recess (72), measured in the median plane of the membrane, being greater than 1 mm,
the porous wall being made of n-doped silicon, and
each pore having a diameter of greater than or equal to 50 nm and less than or equal to 400 nm.

2. Membrane according to Claim 1, having a transmittance of greater than or equal to 10%, or even greater than or equal to 20%, better still greater than or equal to 30%, to any radiation with a wavelength of between 5 µm and 25 µm.

3. Membrane according to either one of Claims 1 and 2, each pore having a diameter of less than or equal to 350 nm.

4. Membrane according to any one of the preceding claims, the pores having a slenderness ratio of greater than or equal to 50, better still greater than or equal to 100.

5. Membrane according to any one of the preceding claims, the pore surface density being between 0.1 µm⁻² and 5.0 µm⁻².

6. Membrane according to any one of the preceding claims, the angle formed by the opposite large faces of the membrane being less than or equal to 5°, the opposite large faces of the membrane being in particular parallel.

7. Membrane according to any one of the preceding claims, at least one, preferably each, of the faces of the membrane exhibiting a surface roughness of less than 20 nm.

8. Membrane according to any one of the preceding claims, at least one part, preferably all, of the area of at least one, preferably of each, of the opposite large faces of the membrane being free of a coating covering it.

9. Process for manufacturing a membrane according to any one of the preceding claims, the process comprising
- the provision of an n-doped silicon substrate which has an electrical resistivity of between 1 Ω.cm and 10 Ω.cm, and of an electrochemical cell comprising an electric generator, a cathode and an anode electrically powered by the electric generator,
the electrochemical cell containing an aqueous electrolyte comprising hydrofluoric acid in a mass concentration of greater than or equal to 10%,
the substrate being placed between the anode and the cathode,
at least one of the faces of the silicon substrate being in contact with the electrolyte,
the cathode and the face of the silicon substrate opposite the cathode, known as the "front face", being in contact with the electrolyte, the front face of the silicon substrate being distant from the cathode,
- electrochemical etching of the substrate by applying with the electric generator a constant electrical voltage of greater than or equal to 10 V between the anode and the cathode, so as to form blind pores in the substrate, each of which has a diameter of greater than or equal to 50 nm and less than or equal to 400 nm and which extend from one face of the substrate to a depth of greater than or equal to 10 µm, the rear face of the substrate being illuminated during the electrochemical etching with light radiation so as to generate charge carriers in the substrate, and
- ablation of part of the substrate so that said pores are open and pass through at least a portion of the substrate entirely across its thickness.

10. Process according to Claim 9, the aqueous electrolyte having a mass concentration of hydrofluoric acid of greater than or equal to 15%, preferably greater than or equal to 30%, better still greater than or equal to 35%.

11. Process according to either one of Claims 9 and 10, comprising, prior to electrochemical etching, illumination of the substrate with light radiation so as to generate charge carriers in the substrate.

12. Process according to any one of Claims 9 to 11, the electrochemical etching being performed until the pores each extend from one face of the support to a depth greater than or equal to 30 µm, preferably greater than or equal to 50 µm, or even greater than or equal to 75 µm.

13. Process according to any one of Claims 9 to 12, the electrochemical etching being performed at a constant electrical voltage of between 25 V and 100 V.

14. Process according to any one of Claims 9 to 13, the substrate having a thickness, prior to electrochemical etching, of between 100 µm and 3 mm, preferably between 500 µm and 1.5 mm.

15. Process according to any one of Claims 9 to 14, the ablation being performed by mechanical polishing, wet chemical etching and/or dry deep etching, the ablation preferably being selective.

16. Process according to any one of Claims 9 to 15, comprising subsequent treatment of the membrane to render the membrane hydrophilic or hydrophobic.

17. Method for characterizing a colony of microorganisms, involving:
- culturing the microorganisms in a nutrient medium, the microorganisms being arranged on one face of a membrane according to any one of Claims 1 to 8 and/or obtained via the process according to any one of Claims 9 to 16, the membrane being in contact with the nutrient medium in such a way that the nutrient medium is transferred via the pores to the microorganisms,
- illuminating the microorganisms in transmission through the membrane by light radiation having at least one wavelength of between 5 µm and 25 µm and acquiring the light radiation that has interacted with the microorganisms.
